(19) Europäisches Patentamt
European Patent Office
Office européen des brevets

(11) EP 1 002 533 B1

(12) # EUROPEAN PATENT SPECIFICATION

(45) Date of publication and mention of the grant of the patent:
**04.12.2002 Bulletin 2002/49**

(51) Int Cl.[7]: **A61K 31/353**, A61P 39/06, A61K 31/35

(21) Application number: **99500138.5**

(22) Date of filing: **10.09.1999**

(54) **Use of chromanes and/or chromenes derivatives to prevent oxidative reactions induced by free radicals**

Verwendung von Chromane und/oder Chromene Derivaten zur Vermeidung von freien Radikalen induzierten oxidativen Reaktionen

Utilisation de derives de chromane et/ou de chromene pour empecher les reactions oxydatives induités par les radicales libre

(84) Designated Contracting States:
**AT BE CH CY DE DK ES FI FR GB GR IE IT LI LU MC NL PT SE**

(30) Priority: **16.09.1998 ES 9801947**

(43) Date of publication of application:
**24.05.2000 Bulletin 2000/21**

(73) Proprietor: **LIPOTEC, S.A.**
**E-08902 L'Hospitalet de Llobregat (Barcelona) (ES)**

(72) Inventors:
- **Pons Lambiez, Fernando**
  **08396 Sant Cebria de Vallalta, Barcelona (ES)**
- **Delgado Gonzalez, Raquel**
  **08032 Barcelona (ES)**
- **Parente Duena, Antonio**
  **08960 Sant Just Desvern (ES)**

(74) Representative: **Davila Baz, Angel**
**c/o Clarke, Modet & Co.,**
**Goya, 11**
**28001 Madrid (ES)**

(56) References cited:
**EP-A- 0 655 239** **WO-A-99/37294**

- **CASAS, JOSEFINA ET AL: "Inhibition of rat liver microsomal lipid peroxidation elicited by simple 2,2-dimethylchromenes and chromans structurally related to precocenes" J. AGRIC. FOOD CHEM. (1992), 40(4), 585-90, XP000877354**
- **IRURRE, JOSEP, JR. ET AL: "Inhibition of rat liver microsomal lipid peroxidation elicited by 2,2-dimethylchromenes and chromans containing fluorinated moieties resistant to cytochrome P-450 metabolism" BIOORG. MED. CHEM. (1993), 1(3), 219-25, XP000892446**
- **MONTOLIU, CARMINA ET AL: "Prevention of glutamate neurotoxicity in cultured neurons by 3,4-dihydro-6-hydroxy-7-methoxy-2,2-dimeth yl-1(2H)-benzopyran (CR-6), a scavenger of nitric oxide" BIOCHEM. PHARMACOL. (1999), 58(2), 255-261, XP000889927**
- **HARMAN D: "ROLE OF FREE RADICALS IN AGING AND DISEASE" ANNALS OF THE NEW YORK ACADEMY OF SCIENCES,US,NEW YORK, NY, vol. 673, 1992, pages 126-141, XP000865672**
- **AMES B N ET AL: "OXIDANTS, ANTIOXIDANTS, AND THE DEGENERATIVE DISEASES OF AGING" PROCEEDINGS OF THE NATIONAL ACADEMY OF SCIENCES OF USA,US,NATIONAL ACADEMY OF SCIENCE. WASHINGTON, vol. 90, no. 17, 1 September 1993 (1993-09-01), pages 7915-7922, XP002912472 ISSN: 0027-8424**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 002 533 B1

Printed by Jouve, 75001 PARIS (FR)

## Description

The present invention is related to the use of anti-oxidants and in particular to the use of chromanes and/or chromenes in oral or topical preparations with the aim of chemically reducing oxidative reactions and those induced by free radicals.

State of the Art

The importance of oxidative reactions or those induced by free radicals is widely known. Different industries such as the cosmetics industry and the food industry, as well as the pharmaceutical industry are continually investigating said processes with the aim of reducing the damaging effects that they produce.

From the cosmetics point of view, given that the skin is always in contact with the air, is constantly exposed to ultraviolet radiation and, inevitably, is subject to environmental factors, it suffers a progressive degradation that can lead to dermatological problems or accelerated ageing.

In the food and pharmaceutical industry the aim lies beyond stabilising the raw materials used. The main objective is to reduce the biological oxidative processes, such as for example reducing the destabilising processes of biological membranes induced by peroxidation of their lipid components of or, also, all those processes induced by peroxynitrite that are generated *in vivo* by the macrophage and neutrophile cells. In fact, peroxynitrite reacts with a large number of biomolecules, such as nitrogenated bases of DNA and the amino acid residues of proteins. Furthermore, it induces lipid peroxidation in cellular membranes and it has been implicated in atherosclerosis, cerebral ischaemia, pulmonar edema, aploplexy, Alhzeimher's disease and tumour genesis.

The authors of the invention previously demonstrated (**US patent 5,605,703**) the potential shown by a series of 2,2-dimethylchromanes and chromenes of general formula A and B (see figure 1) as inhibitors of lipid peroxidation reactions induced by the anti-tumour durg doxorubicine. Similarly, the inclusion of chromane 6 in liposomes that contained said drug led to a significant reduction in the toxicity with i.v. and i.p. administration of doxorubicine encapsulated with respect to the liposomes that did not contain crhomane 6.

Later studies have surprisingly shown that this series of 2,2-dimethylcrhomanes and chromenes show great potential as inhibitors of the nitration reactions induced by peroxynitrite.

Summary of the invention

In view of the state of the art, it is a general object of the present invention to obtain oral or topical preparations that contain a series of 2,2-dimethylchromanes and chromenes with a view to chemically reducing the oxidative reactions or those induced by free radicals, and reducing the damaging effects that these provoke.

Therefore, the invention provides a composition in the form of a gel, emulsion, cream, lotion, tonic or suspension, that can be topically applied or orally administered, and that comprises an effective quantity of a compound of general formula A or B:

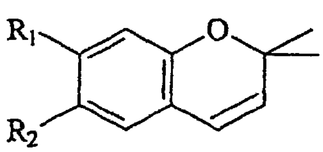

A

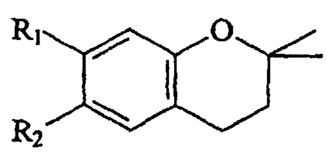

B

wherein R1 and R2 are identical or different and are independently selected from the group consisting of -OH, $-CH_3$, $-CF_3CH_2O-$ and $CH_3O-$ and a pharmaceutically acceptable vehicle.

The composition according to the invention can be applied for pharmaceutical purposes.

Preferably, the composition according to the invention comprises from 0.05 to 20 % of said compound of formula A or B, with respect to total weight of the composition.

The active compounds of formula A or B can be employed in the final composition in direct manner or in a semi-elaborated manner, for example encapsulated in liposomes or in micro-capsules.

Therefore, an additional objective of the invention is to establish procedures for incorporated said compounds effectively into oral and topical preparations.

Brief Description of the Invention

The invention will be described with reference to the attached drawings, in which:

Figure 1 illustrates the formulae of the chromenes (1-4) and chromanes related to the present invention.

Figure 2 shows the increase in lipid peroxidation of liposomes of soybean lecithin as a function of time on being submitted to UVC light in the presence of different anti-oxidant agents.

Figure 3 shows the efficacy of chromanoe6 in the inhibition of the formation of 3-nitrotyrosine induced by peroxynitrite.

Detailed description of the invention

As a first step in the development of the present invention studies have been carried out to establish the potential of 2,2-dimethylchromanes and chromenes with a view to chemically reducing the oxidative reactions or those induced by free radicals, and reducing the damaging effects that these provoke.

In the first study described in the present invention, example 1, the capacity of these compounds for preventing lipid peroxidation induced by UV radiation by means of a, simple but sure, *in vitro* method has been examined. A solutions of phosphatidylcholine liposomes has been used as a target for the peroxidation due to the similarity with biological membranes composed of phospholipid bi-layers. UVC radiation, although it does not get to affect living beings, produces an effect analogous to that of UVE but in a much shorter time span and, for this reason, is routinely used in *in vitro* studies. During exposure to UV light, aliquots are taken that allow the lipid peroxidation induced to be determined by spectrophotometric determination of the increase in substances that react with thiobarbituric acid measured at 532 nm.

The second of the studies, carried out to support the present invention, is described in example 2 and involves the reactions of nitration induced by peroxynitrite. Peroxynitrite is generated *in vivo* by a controlled reaction by means of diffusion between the NO· radical, a biologically very important molecule that acts as a vasodilator, neurotransmitter and immune regulator, and the superoxide radical anion, $O_2\cdot^-$, as is shown in the following equation:

$$NO\cdot + O_2\cdot^- \rightarrow ONOO^-$$

Peroxynitrite and peroxynitrous acid are found in equilibrium ($pK_a = 6.8$), in such a way that in a strongly basic medium the peroxynitrite predominates, a relatively stable species, while at physiological pH, the equilibrium is found displaced to the peroxynitrous acid side. This acid is highly reactive and can follow two types of very quick process: isomerisation to nitric acid, which is found completely dissociated into nitrate and proton, and the formation of a species of radical type with a high oxidative capacity and nitrating capacity that can cause important damage to the biological matrix. The effective inhibition of the reaction between tyrosine and peroxynitrite allows the evaluation of potential candidates to act as blockers of the peroxynitrite action. This reaction carried out at pH 7.4 yields 3-nitrotyrosine as the main product. Detection of 3-nitrotyrosine in cells and biological fluids has been used recently as a biomarker of the formation of peroxynitrite *in vivo* (**FEBS Letters, 1997, 411, 157-160**).

In agreement with the general objective of the invention, assays have been developed of cutaneous irritation and of acute oral toxicity to demonstrate the harmlessness of 2,2-dimethylchromanes and chromenes, examples 3 and 4.

Finally, in examples 5 to 8 solubility studies and different methods of incorporation into different final preparations of 2,2-dimethylchromanes and chromenes with the object of topical or oral administration are described.

The examples that are presented below, are described as a support for the particular aspects of the invention, and should on no account be taken as limiting the scope of the invention.

EXAMPLE 1

Evaluation of lipid peroxidation using liposomes as membrane model

Following the model proposed by **Pelle *et al.*,: Annals of the New York Academy of Sciences (1989) 570, 491-494** with minor modifications, 27 mg of soybean lecithin were weighed out and 1.5 ml of phosphate buffer pH 7.4 (PBS) added. The compounds studied as protectors of lipid peroxidation were added dissolved in ethanol in the quantity necessary for reaching different molar percentages with respect to the lecithin present. In all cases multi-laminar liposomes were obtained by mechanical agitation for one minute.

Once obtained, both the liposomes incorporating anti-oxidants and those that did not incorporate them, were exposed to UVC radiation (254 nm). A sample without anti-oxidants and not exposed to radiation was prepared as a

control. UVC radiation was used because it produces effects similar to those produced by UVB but in much shorter time periods. For example, one hour of UVC radiation would produce effects similar to 7 hours of UVB radiation.

During exposure, aliquots were taken at different times to determine the lipid peroxidation.

*TBARS test*. Lipid peroxidation was carried out by spectrophotometrically determining the increase in production of substances that react with thiobarbituric acid (TBARS). Briefly, 0.2 ml of liposomal suspension, 3 ml of 20% trichloroacetic acid and I ml of 1% thiobarbituric acid were incubated at 100° C for 25 minutes, then centrifuged at 2000 rpm for 10 minutes and the adsorption measured at 532 nm.

As is shown in figure 2, the irradiated liposomes exhibit a greater production of lipid peroxides than the corresponding control not submitted to UVC radiation. Using this model the protective power against lipid peroxidation induced by chromane 6 was compared with respect to α-tocopherol and γ-tocopherol, known lypophilic anti-oxidants. The results indicated that chromane 6 has an inhibitory potential at least 10 times greater than α-tocopherol at the concentrations assayed and of the order of that exhibited by γ-tocopherol.

EXAMPLE 2

Efficacy of chromane 6 in the inhibition of the formation of 3-nitrotyrosine

The reactivity of tyrosine (1 mM) with peroxynitrite (1 mM) was determined in the presence of different concentrations of chromane 6 as is shown in figure 3.

More exactly, the incubations were carried out with 2 ml of a mixture of 0.05 M potassium phosphate buffer (pH = 7.4): methanol 3:1 v/v, kept at 37° C and submitted to vigorous shaking. To each solution that contained 1 mM tyrosine and increasing amounts of chromane 6, a solution of peroxynitrite was added to give the final concentration of this of 1 mM. After 10 minutes, 1 ml of a solution of 4-chlorophenylalanine (2 mM) was added as an internal standard and an extraction with hexane carried out. The aqueous phase obtained from the extraction was analysed by HPLC to determine the amount of tyrosine consumed in the reaction.

EXAMPLE 3

Cutaneous Irritation Assay

Determination of the Primary Irritation Index from dorsal application was carried out according to the method described by the "Journal Officiel de la Republique Française of the 21st of February 1982".

More exactly, the assay was carried out with groups of 3 New Zealand albino rabbits weighing between 1900 and 2000 g. The application was carried out administrating 0.5 ml of a cream that contained 0.5 % of chromane 6 to the dorsal of the animal once it had been shaved. The results determined from observation of the treated animals are presented in table **1**.

Therefore, according to the experimental data obtained an IIC value of 0. Therefore, use of chromane 6 in the conditions described was classified as NON IRRITANT.

TABLE 1

| | | TIME | Rabbit number | | |
|---|---|---|---|---|---|
| | | | 1 | 2 | 3 |
| ERITHEMA | INTACT SKIN | 24 | 0 | 0 | 0 |
| | | 72 | 0 | 0 | 0 |
| EDEMA | SKIN WITH CLEAVES | 24 | 0 | 0 | 0 |
| | | 72 | 0 | 0 | 0 |
| | SUBTOTAL | | | 0 | 0 |
| ERITHEMA | INTACT SKIN | 24 | 0 | 0 | 0 |
| | | 72 | 0 | 0 | 0 |
| EDEMA | SKIN WITH CLEAVES | 24 | 0 | 0 | 0 |
| | | 72 | 0 | 0 | 0 |
| | SUBTOTAL | | | 0 | 0 |

TABLE 1 (continued)

| | | TIME | Rabbit number | |
|---|---|---|---|---|
| TOTAL | | | 0 | 0 |
| INDIVIDUAL INDEX OF PRIMARY IRRITATION | | | 0 | 0 |
| AVERAGE INDEX OF PRIMARY IRRITATION | | | | |

EXAMPLE 4

Assay of acute toxicity after oral administration

For this assay 5 Swiss albino mice weighing approximately 20 g were taken and the quantity of chromane 6 administered, dissolved in polysorbate 80, corresponding to a dosage of 5000 mg/kg of body weight. The animals were maintained in standard establuation and an evaluation carried out by observing the animals for 14 days after administration. The results indicate that there were no changes in the behaviour of the animals and that no death in the treated group occurred. Therefore, it could be concluded that the oral administration of chromane 6 at a dosage of 5000 mg/kg body weight did not show any toxicity.

EXAMPLE 5

Assays of Solubility of Chromane 6

Assays of solubility of chromane 6 were carried out using various solvents as a support for the invention for the later application in preparations of products for topical and oral administration. Two concentrations of solution of chromane 6 were assayed (2 and 5 %) by agitation at 65° C in the solvents that are indicated in table 2. After sufficient time had passed to allow the mixture to reach 25° C it was observed whether undissolved product was present or not.

TABLE 2

| | CHROMANE 2 % | | CHROMANE 2 % | |
|---|---|---|---|---|
| SOLVENT | 65° C | 25° C | 65° C | 25° C |
| Ethanol | Soluble | Soluble | Soluble | Soluble |
| Butylglycol | Soluble | Soluble | Soluble | Soluble |
| Isopropanol | Soluble | Soluble | Soluble | Soluble |
| Butandiol | n.a. | n.a. | Soluble | Precipitates |
| Glycerine | Insoluble | Insoluble | n.a. | n.a. |
| Propylenglycol | Soluble | Soluble | Soluble | Soluble |
| PEG-200 | Soluble | Soluble | Soluble | Soluble |
| PEG-400 | Soluble | Soluble | Soluble | Soluble |
| Wheatgerm oil | Soluble | Soluble | Soluble | Precipitates |
| Mineral oil | Insoluble | Insoluble | n.a. | n.a. |
| Myritol 318® | Soluble | Soluble | Soluble | Soluble |
| Octyl cocoate | Soluble | Soluble | Soluble | Precipitates |
| Polysorbate 20 | Soluble | Soluble | Soluble | Soluble |
| Polysorbate 80 | Soluble | Soluble | Soluble | Soluble |
| n.d.- Not determined | | | | |

EXAMPLE 6

Use of chromane 6 in preparations for topical use

a) Cream (w/o) with protection from UVB light

For the preparation of 1000 g of cream, raw materials were weighed to make up the relative percentages shown in table 3.

TABLE 3

| | | Quantity in percent |
|---|---|---|
| A | Octyl cocoate | 20.000 |
| | Eusolex 6300™ | 2.000 |
| | Beeswax | 0.500 |
| | Sorbtan sesquioleate | 3.000 |
| | Paraffin | 1.000 |
| | Chromane 6 | 0.100 |
| B | Sulphate monohydrate | 0.500 |
| | Borax | 0.035 |
| | Phenonip™ | 0.500 |
| | Demineralised water | Sufficient quantity for 100 % |
| C | Perfume | 0.100 |

Compounds of A and B were mixed separately at 70° C.
Once two homogeneous solutions were obtained, part B was added slowly to part A with turbine stirring. After 10 minutes the mixture is cooled under gentle stirring until room temperature is reached and the perfume is added.

b) Sun oil with protection from UVB light

For the preparation of 1000 g of oil, the necessary raw materials were weighed to give the relative percentages shown in table 4.

TABLE 4

| | Quantity present in percent |
|---|---|
| Eusolex 6300™ | 2.000 |
| Isopropyl myristate | 10.000 |
| Chromane 6 | 0.100 |
| Octyl cocoate | 20.000 |
| Perfume | 0.100 |
| Low viscosity paraffin oil | s.q.for 100 % |

Separately, the Eusolex 6300™ is dissolved with the isopropyl myristate and chromane 6 with the octyl cocoate. To the paraffin oil the previously obtained mixtures and the perfume are added slowly with gentle stirring.

c) Hydro-alcohol gel with UVB protection

For the preparation of 1000 g of gel, the necessary raw materials are weighed to give the relative percentages shown in table 5.

TABLE 5

| | | Quantity present in percent |
|---|---|---|
| A | Eurolex 232™ | 2.000 |
| | Aliantion | 0.200 |
| | Carbopol 940™ | 1.500 |
| | Triethanolamine | 4.000 |
| | phenonip™ | 1.000 |
| | Demineralised water | s.q.f 100 % |
| B | Ethanol | 30.000 |
| | Chromane 6 | 0.500 |
| | perfume | 0.100 |

For the preparation of the gel the Eusolex™ is dissolved in all the water by the addition of triethanolamine. Then, the alantoin is dissolved and the Carbopol™ dispersed. Once the carbomer has been hydrated solution B is added and the pH adjusted if necessary.

EXAMPLE 7

Use of chromane 6 in preparations for oral use

a) Energetic drink with anti-radical properties

For the preparation of 1000 ml of drink, the necessary raw materials are weighed according to the relative percentages presented in table 5

TABLE 5

| | | Quantity in percent |
|---|---|---|
| A | Soy lecithin | 1.000 |
| | Soy oil | 4.000 |
| | $Na_2HPO_4$ | 1.150 |
| | $Na_2H_2PO_4 \cdot 2H_2O$ | 0.280 |
| | NaCl | 0.280 |
| | Sodium benzoate | 0.100 |
| | Sodium sorbate | 0.100 |
| | Chromane 6 | 0.500 |
| | Glucose | 1.000 |
| | Water | s.q.f. 100 % |
| B | NaOH | s.q.f pH 7.4 |

For the preparation of the drink the chromane 6 is dissolved in soy oil. The lecithin is dissolved in the mixture obtained and then the mixture added to the solution obtained from the rest of the components adjusted to pH 7.4, while maintaining vigorous stirring. Finally, the suspension is submitted to a micro-fluidation process to obtain a homogeneous preparation with small particles sizes.

b) Preparation of milli-capsules with anti-radical properties

For the preparation of the drink according to Spanish patent P9602470, in the first place the milli-capsules

are obtained, the relative compositions are shown in table 6.

TABLE 6

| | Quantity in percent |
|---|---|
| Soybean oil | 10.000 |
| Chromane 6 | 1.000 |
| Agar-agar | 1.000 |
| Sodium glutinate | 0.600 |
| Sodium benzoate | 0.300 |
| Titanium oxide | 0.100 |
| glycerine | 5.000 |
| water | s.q.f. 100 |

For the preparation of the milli-capsules the sodium glutinate and agar-agar are dissolved, heating the system of 95° C. Chromane is added dissolved in soybean oil to the previously obtained solution, adding the benzoate as a preservative and the titanium oxide colorant dispersed in glycerine.

The resulting solution is added drop by drop through an orifice of 0.5 mm to a solution of 0.3 % $Ca_2Cl$ in water. The milli-spheres obtained are recovered by filtration and the $Ca_2Cl$ washed with distilled water.

To obtain the drink with anti-radical properties the necessary raw materials to give the following relative percentages given in table 7 are weighed out.

TABLE 7

| | | Quantity in percent |
|---|---|---|
| A | Kappa ADG39 type carragenine | 0.042 |
| | Garrofin gum | 0.060 |
| | LM35 type aminated pectin | 0.092 |
| | Potassium chloride (Hercula type 104 AS) | 0.080 |
| | Sodium bicarbonate | 0.020 |
| | Sodium hexamethaphosphate | 0.012 |
| | Dextrose | 0.096 |
| H | Milli-spheres | 2.000 |
| | Concentrated syrup containing: | 80.000 |
| | Aromas, colorants and sweetener-water | q.s.f. 100 |

The following procedure is followed in a suitable recipient:

1.- The mixture of ingredients A is dispersed in de-mineralised water stirring strongly
2.- The solution from step 1 is heated until boiling
3.- This solution is cooled to 50° C, thus obtaining what we will call the carrageenate solution.
4.- The carrageenate solution is mixed with they concentrated drink syrup, and the resulting solution cooled to 20° C.
5.- The milli-spheres obtained earlier are added and dispersed to give the final form of the drink.

c) Preparation of lyophilised liposome capsules incorporating chromane 6

According to that described in Spanish patent P9700073, in 200 ml of chloroform/methanol (75:25) a mixture composed of 1244.2 mg of hydrogenated egg lecithin, 499.7 mg of cholesterol and 175 mg of chromane 6 were dissolved. The organic solvents were then eliminated by rotary evaporation, and remaining traces of solvent removed by passing $N_2$ through the mixture or by lyophilisation. The lipid film thus obtained was hydrated with 50 ml of NaCl solution

at 0.9 % by stirring in a vibromixer at intervals of 30 seconds, followed by leaving in a bath kept at 60° C for the same time until an effective stirring time of 10 minutes is reached. In this way the lyposomal suspension was obtained.

The resulting lyposomal suspension was frozen or lyophilised to obtain a lyophilised powder which was introduced into hard gelatine capsules (50 mg of lyophilate in each gelatine capsule). The resulting capsules were coated with a gastro-protector (EUDRAGIT-L)™ by repeated immersion in a solution of the gastro-protector polymer in isopropanol and then drying in an air current.

## Claims

1. Use of a compound of general formula A or B:

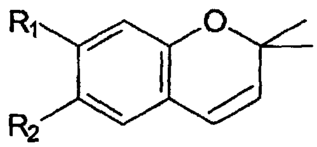

A

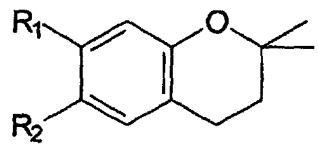

B

wherein R1 and R2 are identical or different and are independently selected from the group consisting of -OH, $-CH_3$, $-CF_3CH_2O$- and $CH_3O$-, in the manufacture of a pharmaceutical composition for the oral or topical treatment of diseases associated with oxidative reactions or reactions induced by free radicals.

2. The use according to claim 1, **characterised in that** the compounds of general formula A or B are in concentrations that range from 0.05 % to 20 % with respect to total weight.

3. The use according to any of claims 1 and 2, **characterised in that** the compounds of general formula A or B are used either directly or semi-elaborated.

4. The use according to claim 3, **characterised in that** the compounds of general formula A or B are used encapsulated in liposomes or in micro-capsules.

5. The use according to any of claims 1 to 4, **characterised in that** the pharmaceutical composition for topical treatment is a gel, emulsion, cream, lotion, tonic or suspension.

## Patentansprüche

1. Verwendung einer Verbindung der allgemeinen Formel A oder B:

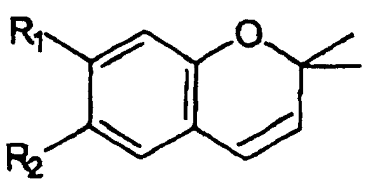

A

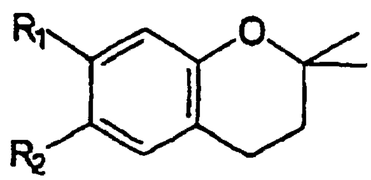

B

wobei R1 und R2 identisch oder verschieden sind und unabhängig voneinander aus der aus -OH, $-CH_3$, $-CF_3CH_20$- und $CH_30$- bestehenden Gruppe ausgewählt sind, bei der Herstellung einer pharmazeutischen Zusammensetzung für die orale oder lokale Behandlung von mit oxidativen Reaktionen oder durch freie Radikale induzierten Reaktionen zusammenhängenden Krankheiten.

2. Verwendung nach Anspruch 1, **dadurch gekennzeichnet, dass** sich die Verbindungen der allgemeinen Formel

A oder B in Konzentrationen vorliegen, die zwischen 0,05 % und 20 % bezogen auf das Gesamtgewicht liegen.

3. Verwendung nach Anspruch 1, **dadurch gekennzeichnet, dass** die Verbindungen der allgemeinen Formel A oder B entweder direkt oder halb verarbeitet verwendet werden.

4. Verwendung nach Anspruch 3, **dadurch gekennzeichnet, dass** die Verbindungen der allgemeinen Formel A oder B in Liposomen oder in Mikrokapseln eingekapselt sind.

5. Verwendung nach einem der Ansprüche 1 bis 4, **dadurch gekennzeichnet, dass** die pharmazeutische Zusammensetzung zur lokalen Behandlung ein Gel, eine Emulsion, eine Creme, eine Lotion, ein Tonikum oder eine Suspension ist.

**Revendications**

1. Utilisation d'un composé de formule générale A ou B :

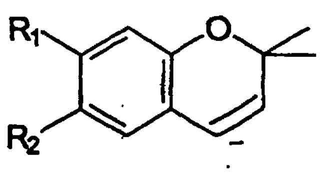

A

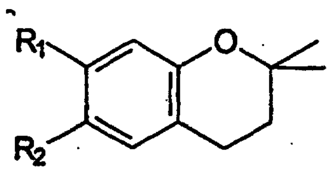

B

dans lesquelles $R_1$ et $R_2$ sont identiques ou différents et sont indépendamment choisis dans le groupe constitué par -OH, $-CH_3$, $-CF_3CH_2O-$ et $CH_3O-$, dans la fabrication d'une composition pharmaceutique pour le traitement oral ou topique de maladies associées à des réactions oxydatives ou des réactions induites par des radicaux libres.

2. Utilisation selon la revendication 1, **caractérisée en ce que** les composés de formule générale A ou B sont à des concentrations qui vont de 0,05 % à 20 % par rapport au poids total.

3. Utilisation selon l'une quelconque des revendications 1 et 2, **caractérisée en ce que** les composés de formule générale A ou B sont utilisés soit directement soit semi-élaborés.

4. Utilisation selon la revendication 3, **caractérisée en ce que** les composés de formule générale A ou B sont utilisés encapsulés dans des liposomes ou dans des micro-capsules.

5. Utilisation selon l'une quelconque des revendications 1 à 4, **caractérisée en ce que** la composition pharmaceutique pour le traitement topique est un gel, une émulsion, une crème, une lotion, un tonique ou une suspension.

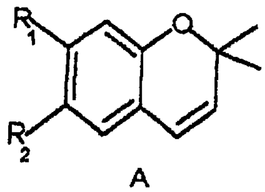

A

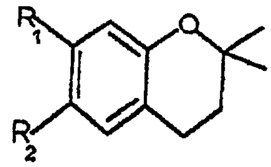

B

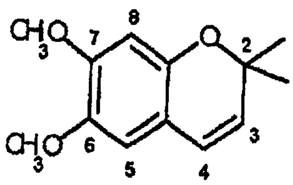

1

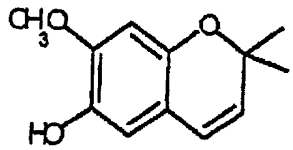

2

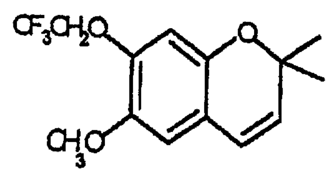

3

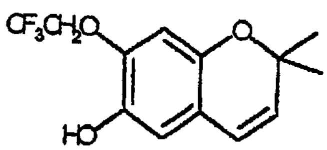

4

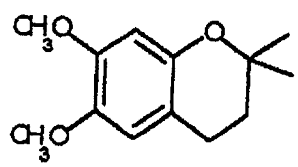

5

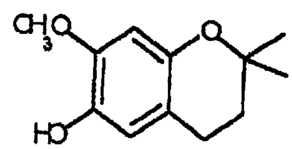

6

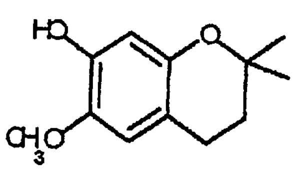

7

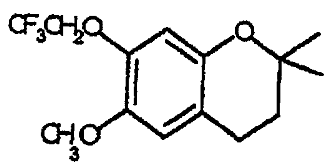

8

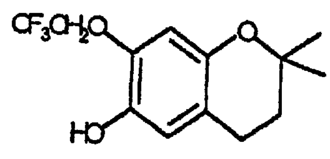

9

Fig. 1

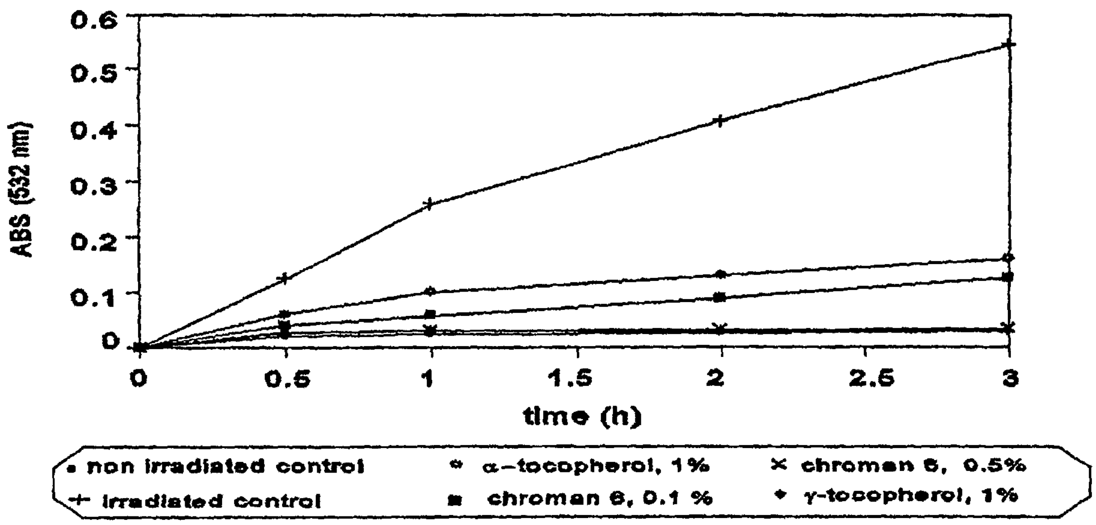
0.6
0.5
0.4
0.3
0.2
0.1
0
ABS (532 nm)
0
0.5
1
1.5
2
2.5
3
time (h)
non irradiated control
irradiated control
α-tocopherol, 1%
chroman 6, 0.1 %
chroman 6, 0.5%
γ-tocopherol, 1%

Fig.2

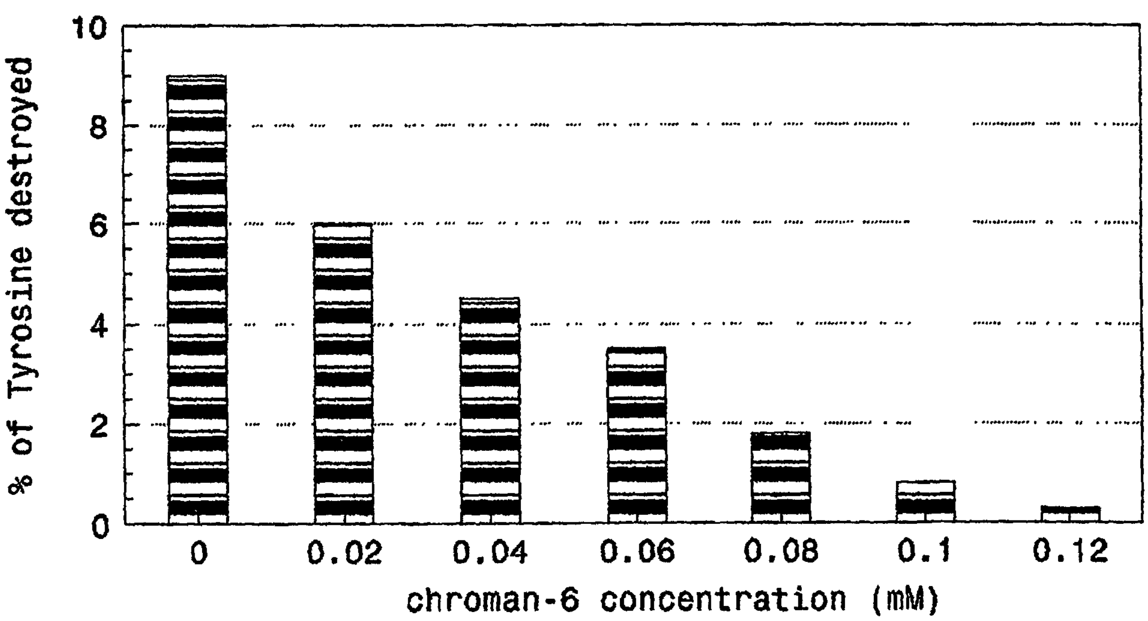
10
8
6
4
2
0
% of Tyrosine destroyed
0
0.02
0.04
0.06
0.08
0.1
0.12
chroman-6 concentration (mM)

Fig. 3